# EUROPEAN PATENT APPLICATION

(11) **EP 1 159 979 A2**
(43) Date of publication of application: **05.12.2001**
(21) Application number: 01110954.3
(22) Date of filing: 07.05.2001
(51) Int. Cl.: A61M 5/32

(54) **Shieldable needle assembly**

(30) Priority: 31.05.2000 US 584452
(71) Applicant: Becton, Dickinson and Company, Franklin Lakes, New Jersey (US)
(72) Inventor: Cipoletti, Robert K., Pompton Plains, New Jersey 07444 (US); Goldstein, Charles, Chapel Hill, North Carolina 27514 (US)
(74) Representative: Ruffles, Graham Keith

(57) **Abstract**

A shieldable needle assembly includes an elongate needle (12) with proximal and distal ends and a bore (20), a hub (22) fixedly attached at the proximal needle end for attaching to a fluid handling device (24) so that the needle bore is in fluid communication with the fluid handling device, and a guard (26) slidably movable on the needle between a proximal position wherein the distal needle end is exposed and a distal position wherein the distal needle end is protected. The guard has a passageway forming a fluid tight seal about the needle surface, and an attachment fitting (32) so that when the guard is in the distal position, if a device with a conjugate fitting is releasably attached to the guard, the fitting is in fluid communication with the fluid handling device. The assembly also has a hinged arm (38) articulated to the hub and the guard for movement between the proximal and distal positions.

## Description

Field of Invention: The present invention is generally related to medical devices and more particularly to hypodermic needle assemblies having a needle that is selectively exposed and covered by a practitioner.

### Background:

In the medical device field, many devices are designed to provide a practitioner with access through the skin of a patient. In order to provide this access, the devices often incorporate some sort of "sharp". Exemplary of these devices are scalpels, hypodermic syringe needles, catheter placement needles and the like. Both catheter placement needles and hypodermic syringe needles are necessarily elongate, slender and very sharp. Most of these needles are intended for only a particular use, and once used for their intended purpose are considered hazardous to anyone who may have reason to encounter them, including the practitioner who is using the device, those assisting the practitioner and any support staff who may be involved in the ultimate disposal of the devices. The hazards related to used "sharps" medical devices are well reported and need not be further dwelt upon here.

There are many products, both patented and unpatented, that are available to practitioners and support staff to assist in the handling and disposal of such sharps. Initial efforts to protect practitioners and support staff from used needles included devices to cut off needles, various kinds of receptacles for receiving and disposing of sharps. More recently, specialized shielding devices and retractable needles have been disclosed. While many of these specialized shielding devices provide practitioners and support staff with protection from inadvertent exposure to sharps, many of the available devices impose restrictions upon the ability of the practitioner to use the device for some procedures, particularly sequential procedures, as many of the devices, once activated are not selectively reversible.

A disclosure by Sweeney, et al., U.S. Patent No. 5,348,544, teaches a safety shield for a needle that includes a guard that is slidably movable along the needle cannula from a proximal position where the tip of the needle is exposed to a distal position where the needle cannula is safety shielded. In a preferred embodiment of the disclosed device, the guard includes a spring clip that substantially prevents the guard from being retracted to expose the point of the needle after it has been deployed.

A disclosure by Jenkins, et al. describes a protector with a cap that is movable in use along the implement between an inoperative position in which it exposes the pointed end or sharp edge portion of the implement an operative position in which it covers the pointed end or sharp edge portion of the implement.

Another disclosure by Adwers, et al., U.S. Patent No. 5,735,827, teaches a shield assembly that includes an inner shield and an outer shield. The inner shield includes a guard that is selectively movable between a proximal position which permits use of the needle cannula and a distal position which safely covers the tip of the needle cannula. When the use of the device is completed, the outer shield may be replaced on the device to substantially irreversibly latch the outer shield over the cannula to preclude reuse of the needle.

Ciazza, et al., U.S. Patent No. 5,738,665, discloses a shieldable needle assembly that includes a needle guard slidably movable along a needle cannula from a proximal position where the distal end of the needle cannula is exposed to a distal position where the distal end of the needle cannula is safely shielded within the needle guard. The device disclosed in this patent includes a hinged arm assembly that connects the needle guard assembly to the needle hub. There is a trigger that is articulated to the needle hub at a location proximal to the hinged arm that the practitioner applies finger pressure to for urging the hinged arm from a collapsed position to an extended position.

Ciazza, et al., U.S. Patent No. 5,910,130, discloses a shieldable needle assembly that includes a needle guard slidably movable along a needle cannula from a proximal position where the distal end of the needle cannula is exposed to a distal position where the distal end of the needle is safely shielded within the needle guard. There is a hinged arm assembly that connects the needle guard assembly to the needle hub with a latch mechanism for latching the arm in the extended position to substantially prevent retraction of the needle guard from the distal position on the needle.

U.S. Patent No. 5,925,020 to Nestell discloses a needle point barrier that has a resiliently deflectable spring arm, a link arm and a barrier arm. The proximal end of the spring arm is secured near the proximal end of the needle. The link arm has one end hingedly connected to the spring arm and an opposed end hingedly connected to a proximal position on the barrier arm. The barrier arm can be urged distally by force applied to the proximal end of the barrier arm. the barrier arm when moved distally moves to a position where the distal end of the needle is shielded.

While all of these disclosures provide practitioners with devices that are useful to shield the distal point of a needle, none of the teachings suggest that the needle could be shielded with a connector suitable for use with fluid transfer devices. Such a device is disclosed hereinbelow.

### Summary

A shieldable needle assembly of the present invention includes an elongate needle with a proximal and a distal end that has an outside surface and a hollow bore therethrough. The assembly has a hub fixedly attached at the proximal end of the needle for attaching the assembly to a fluid handling device so that the bore of the needle is in fluid communication with the fluid handling device. The assembly has a guard disposed for selective slidable movement on the needle between a proximal position wherein the distal end of the needle is exposed and a distal position wherein the distal end of the needle is substantially protected from inadvertent exposure. The guard has a passageway therethrough sized to form a substantially fluid tight seal about the outside surface of the needle. The guard also includes an attachment fitting so that when the guard is in the distal position, with the distal end of the needle being substantially protected from inadvertent exposure, if a device with a conjugate fitting is releasably attached to the guard, the fitting is in fluid communication with the fluid handling device attached to the hub. The assembly also has a hinged arm articulated to the hub and to the guard for defining a movement between the proximal position and the distal position.

The shieldable needle assembly of the invention provides practitioners with the ability to have a sharp needle for some aspects of a sequential procedure and an alternate fitting, i.e., the attachment fitting on the guard, selectively available. Conventional shielding devices do not provide for practitioner's needs to selectively switch between one type of device and another when using fluid handling devices. Often, a shielding device, once activated is irreversible. In many procedures involving fluid transfers, the practitioner uses a sharpened needle, then may need a blunt cannula or similar and subsequently again require the sharpened cannula. As another example, a medicament dosage may be prepared using a syringe or other fluid handling device at a location remote from the patient and then need to be transported to the patient and administered to the patient either as a direct injection, an injection into a catheter or into an intravenous set. In many cases, the practitioner may not necessarily have the exact mating fitting available at the patient's bedside. The shielded needle assembly of the invention allows the practitioner to have the protection of a shielded needle as well as a choice of another fitting available in one device.

### Brief Description of the Drawings

Fig. 1 is a partially exploded view of the shielding device of the invention with the distal portion of the needle exposed;
Fig. 2 is a perspective view of the shielding device of Fig. 1 mounted on a syringe;
Fig. 3 is a perspective view of the shielding device of Fig. 1 mounted on a syringe and extended to shield the distal portion of the needle;
Fig. 4 is a schematic cross-sectional view of the shielding device of the invention taken from Fig. 2 along the line 4-4;
Fig 5 is a schematic cross-sectional view of the shielding device of the invention taken from Fig. 3 along the line 5-5;
Fig. 6 is a perspective view of the shielding device of the invention with the guard portion in the proximal position and the needle portion in use;
Fig. 7 is a perspective view shielding device of the invention with the guard in the distal position and with a catheter removably attached to the mounted on the shield portion; and
Fig. 8 is a perspective view of an alternate embodiment of the shielding device of the invention.

### Detailed Description

While this invention is satisfied by embodiments in many different forms, there are shown in the drawings and herein described in detail, embodiments of the invention with the understanding that the present disclosure to be considered as exemplary of the principles of the present invention and is not intended to limit the scope of the invention to the embodiments illustrated. The scope of the invention is measured by the appended claims and the equivalents.

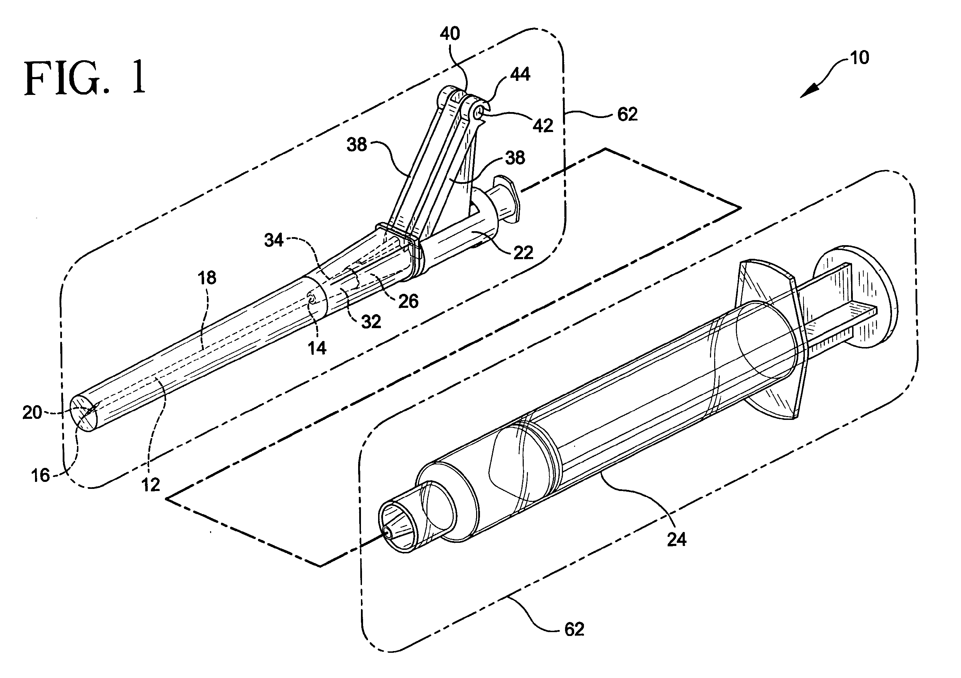

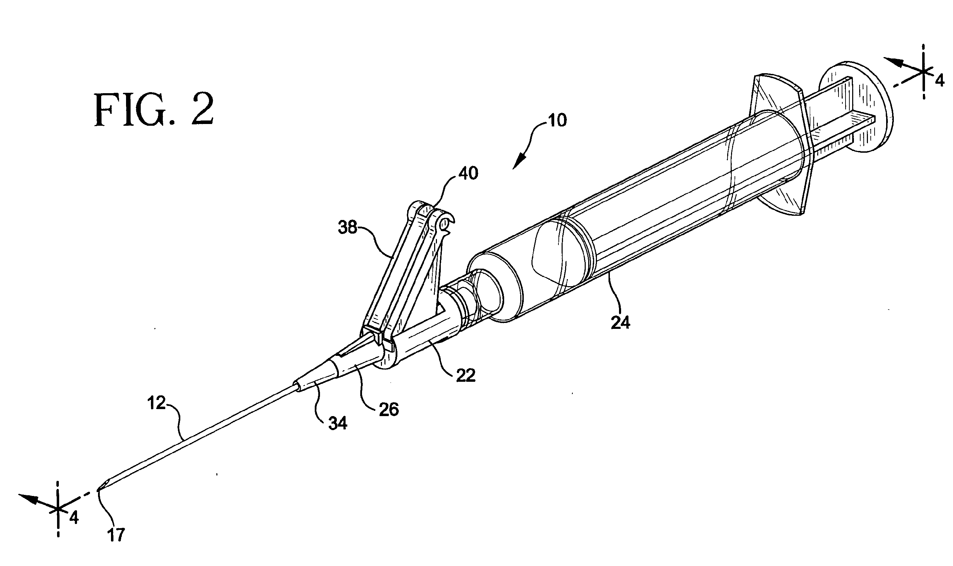

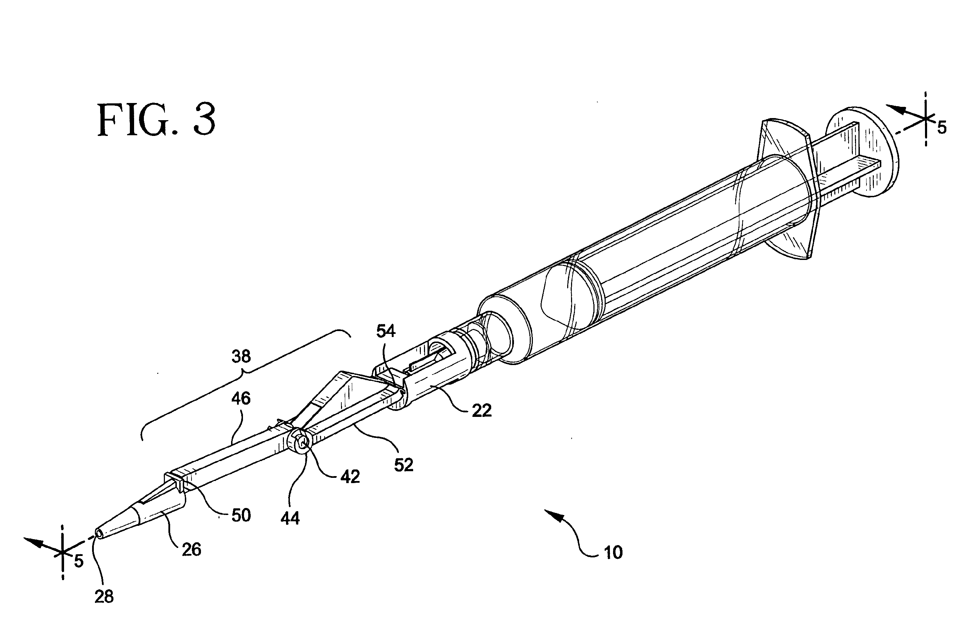

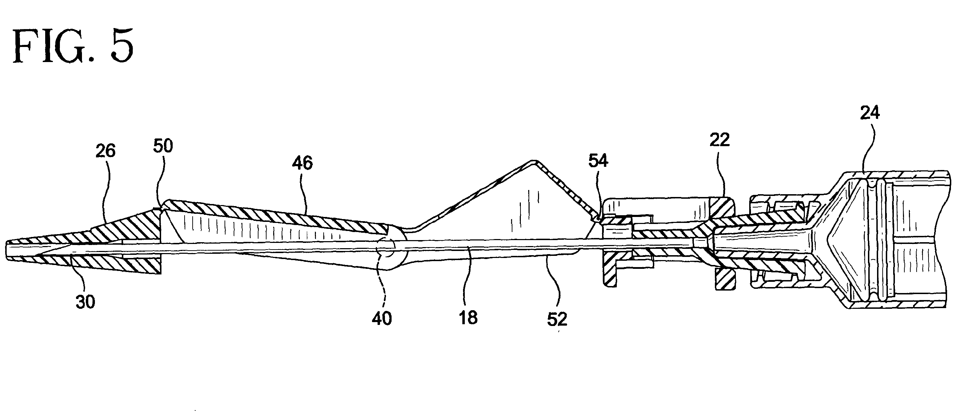

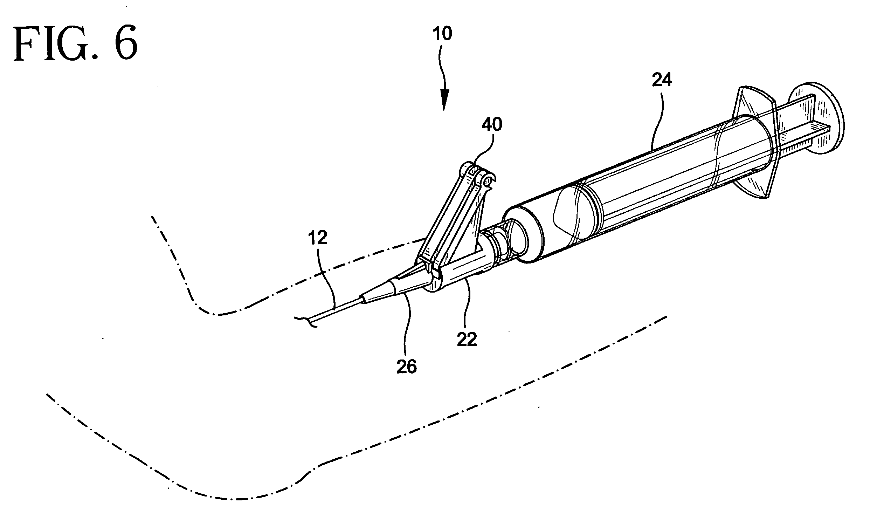

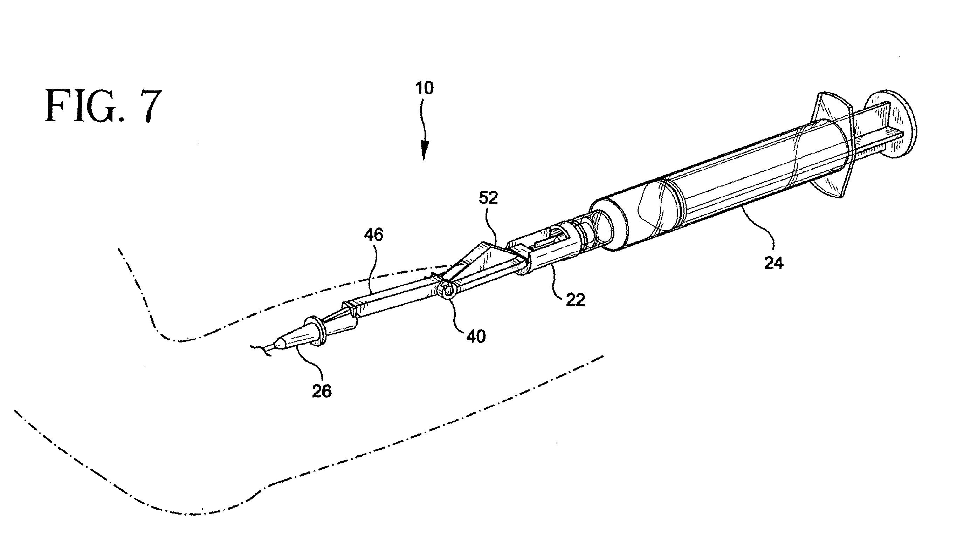

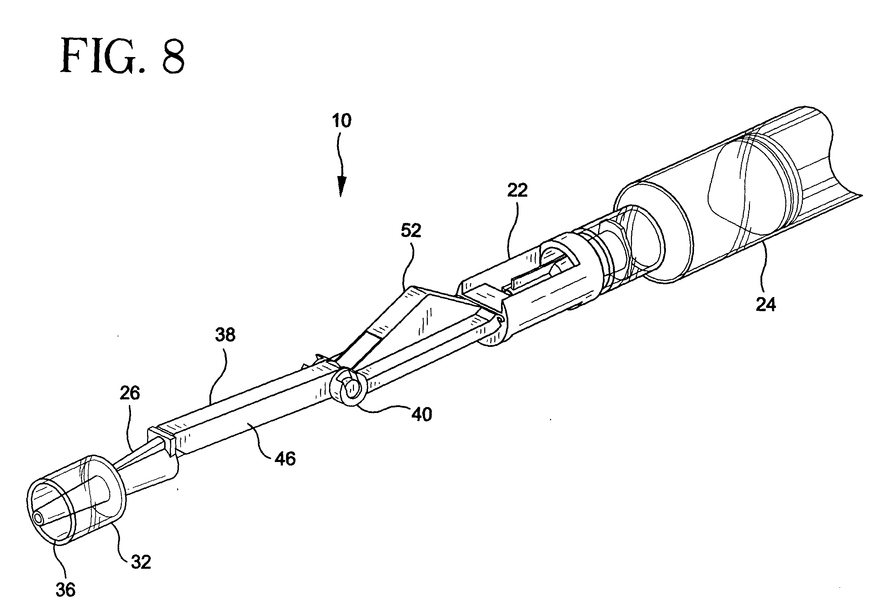

Referring to Figs. 1-8, a shieldable needle assembly 10 of the invention includes an elongate needle 12 with a proximal end 14 and a distal end 16. Needle 12 has an outside surface 18 and a hollow bore 20 therethrough. Assembly 10 has a hub 22 fixedly attached at proximal end 14 of needle 12 for attaching assembly 10 to a fluid handling device, such as a syringe 24 and the like, so that bore 20 of needle 12 is in fluid communication with the fluid handling device. The assembly further includes a guard 26 disposed for selective slidable movement on needle 12 between a proximal position, best seen in Figs. 1, 2, 4 and 6 wherein distal end 16 of needle 12 is exposed and a distal position, best seen in Figs. 3, 5, 7 and 8 wherein distal end 16 of the needle is substantially protected from inadvertent exposure. Preferably, distal end 16 of the needle is formed into a sharpened point 17. Guard 26 has a passageway 28 therethrough with an inside diameter "d" that is sized to form a substantially fluid tight seal 30 about outside surface 18 of the needle. Guard 26 further includes either an attachment 32 such as a luer slip fitting 34, Fig. 1, a threaded luer fitting (not shown), a blunt cannula 36, Fig. 8, for penetrating a slit septum or other attachment fittings so that when guard 26 is in distal position, a conjugate attachment releasably attached to guard 26 is in fluid communication with the fluid handling device attached to hub 22, best seen in Fig. 7. Assembly 10 also includes a hinged arm 38 articulated to hub 22 and to guard 26 for defining the movement between the proximal position and the distal position.

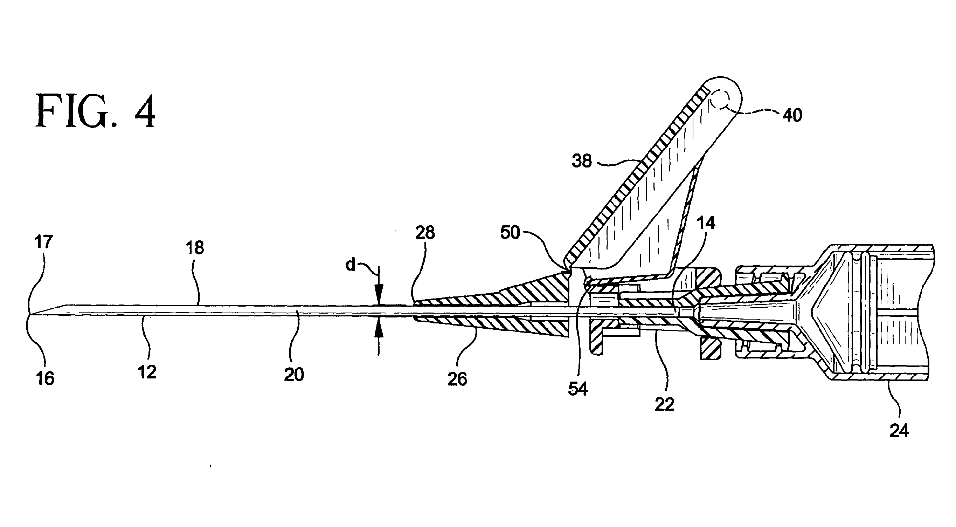

Referring to Figs. 1 and 4, arm 38 includes a first hinge 40 which may include from a peg 42 and socket 44 as shown in Fig. 1 or other suitable hinges. Preferably, guard 26 is formed with a distal portion 46 of arm 38 as a single article of manufacture with an attachment 50 between arm 38 and guard 26 in the form of a living hinge. Preferably, hinge 40 is located between distal portion 46 and proximal portion 52 of arm 38 so that guard 26 is selectively retained in either of the proximal position or the distal position by a selective action of the practitioner. Preferably, hub 22 and proximal portion 52 of arm 38 are formed as a single article of manufacture with an attachment 54 in the form of another living hinge. The formation of guard 26 and distal portion 46 as a single article and the formation of hub 22 and proximal portion 52 as a single article greatly simplifies the manufacture and assembly of the device 10 by reducing the number of parts. A living hinge is formed by injection molding of the parts from a thermoplastic material, the living hinge portion is a section of reduced thickness compared to the thickness the rest of the part. During the filling of the mold cavity used to form the part, the section of reduced thickness causes some orientation of molecules of the molten thermoplastic. Then, as the thermoplastic material cools from the molten state to a temperature below the melting point temperature, the mold cavity is opened and the part is removed. The part is then immediately flexed at the section of reduced thickness which further orients the thermoplastic molecules and forms the living hinge which then may be repeatedly flexed. Suitable materials for forming guard 26 and hub 22 with their preferably attached arm portions include, but are not limited to, thermoplastic resins such as polypropylene, polyethylene, polycarbonate, polyamides, polyesters and copolymers of polypropylene and polyethylene. In selecting materials for forming the shield portion, preferably, the material selected allows a degree of resiliency and is formed into a shape that sufficient compression of the fitting so that when a conjugate fitting is attached to attachment 32 diameter "d" is reduced. If guard 26 is sufficiently resilient, passageway 28 is preferably somewhat compressed when the fitting is attached so that the contact between needle outside surface 18 and the passageway is maintained in a substantially fluid tight relationship while the fitting is attached and when the fitting is removed, sufficient clearance is provide between the guard passageway and the needle surface to allow the slidable movement of the guard between the proximal and the distal positions.

Preferably, assembly 10 includes a removable shield 60, shown in Fig. 1, disposed to protect the distal point 16 of the needle during shipping and handling. Preferably, assembly 10 is sealed in a package 62 (indicated in phantom in Fig. 1) formed from materials substantially resistant to the passage of microorganisms and exposed to conditions suitable for rendering any microorganisms in package 62 substantially non-viable. When packaged and treated as described above assembly 10 may be considered "sterile" as long as package 62 remains intact. Suitable materials for forming package 62 include, but are not limited to paper, polymeric film, metallic foils, non-wovens as well as composites and combinations of these materials. Suitable conditions for rendering microorganisms include, but are not limited to exposure to chemical agents such as ethylene oxide and vapor phase hydrogen peroxide and exposure to ionizing radiation such as gamma, electron beam and U.V. When materials are selected for forming assembly 10 and package 62, consideration should be given to the sterilization conditions to ensure that the materials selected are compatible with the sterilization conditions.

Assembly 10 of the invention provides practitioners with a need for shielding a medical sharp that is used in a sequential procedure that may require multiple exposures of the sharp with a device that does not impede the usage of the sharp in normal procedures. The assembly of the invention allows the practitioner to use the sharp for one or more parts of a sequential procedure and further allows attachment and detachment of an additional fluid handling device without an additional adapter or removal of the needle, thus providing the practitioner with greater flexibility and convenience in sequential procedures, particularly where the fluid handling device is filled at a location remote from the point of usage.

## Claims

1. A shieldable needle assembly comprising
an elongate needle having a proximal and a distal end, a outside surface and a hollow bore therethrough;
a hub fixedly attached at said proximal end of said needle for attaching said assembly to a fluid handling device so that said bore of said needle is in fluid communication with said fluid handling device;
a guard disposed for selective slidable movement on said needle between a proximal position wherein said distal end of said needle is exposed and a distal position wherein said distal end of said needle is substantially protected from inadvertent exposure, said guard having a passageway therethrough having an inside diameter sized to form a substantially fluid tight seal about said outside surface of said needle, said guard comprising attachment means so that when said guard is in said distal position, wherein said distal end of said needle is substantially protected from inadvertent exposure, a conjugate attachment releasably attached to said guard is in fluid communication with said fluid handling device attached to said hub; and
a hinged arm articulated to said hub and to said guard for defining a movement of said guard between said proximal position and said distal position.

2. The shieldable needle assembly of Claim 1 wherein said attachment means is an attachment selected from the group consisting of a luer slip fitting, a luer lock fitting and a blunt cannula fitting for penetrating a slit septum.

3. The shieldable needle assembly of Claim 1 wherein said hinged arm disposed on said hub and said guard is disposed to selectively retain said guard in said proximal and said distal positions, said guard being movable between said positions by a positive action of a practitioner.

4. The shieldable needle assembly of Claim 1 wherein said distal point of said needle further comprises a sharpened point.

5. The shieldable needle assembly of Claim 1 wherein said hub is formed from a thermoplastic material selected from the group consisting of polyethylene, polypropylene, polycarbonate, polystyrene, polyamide and polyester and copolymers thereof.

6. The shieldable needle assembly of Claim 1 wherein said guard is formed from a thermoplastic material selected from the group consisting of polyethylene, polypropylene, polycarbonate, polystyrene, polyamide and polyester and copolymers thereof.

7. The shieldable needle assembly of Claim 6 wherein said guard is formed from a thermoplastic material having sufficient resiliency so that when a device having a fitting conjugate to said attachment means on said guard is attached to said guard, said inside diameter of said passageway through said is sufficiently reduced to form said substantially fluid tight seal between said guard and said outside surface of said needle.

8. The shieldable needle assembly of Claim 1 wherein said hub further comprises a female luer fitting.

9. The shieldable needle assembly of Claim 1 wherein said hub is fixedly attached to a fluid handling device.

10. The shieldable needle assembly of Claim 9 wherein said fluid handling device is a syringe.

11. The shieldable needle assembly of Claim 1 having been sealed in a package formed from materials substantially resistant to the passage of microorganisms and exposed to conditions that substantially render any microorganisms therein substantially non-viable.
